# EUROPÄISCHE PATENTSCHRIFT

(11) **EP 1 737 422 B1**
(45) Veröffentlichungstag und Bekanntmachung des Hinweises auf die Patenterteilung: **24.02.2010**
(21) Anmeldenummer: 05744657.7
(22) Anmeldetag: 22.04.2005
(51) Int. Cl.: A61K 8/25, A61Q 15/00

(54) **SCHWEISSABSORBIERENDES KOSMETISCHES PRODUKT UND VERFAHREN ZU SEINER HERSTELLUNG**
PERSPIRATION-ABSORBENT COSMETIC PRODUCT AND METHOD FOR THE PRODUCTION THEREOF
PRODUIT COSMETIQUE ABSORBANT LA SUEUR ET SON PROCEDE DE FABRICATION

(30) Priorität: 22.04.2004 DE 102004020646
(43) Veröffentlichungstag der Anmeldung: 03.01.2007
(73) Patentinhaber: Coty B.V., 2031 CC Haarlem (NL)
(72) Erfinder: HWANG, Donna Hui-Ing, Leonia, New York 07605 (US); MACCHIO, Ralph, Sparta, New York 07971 (US); CERNASOV, Domnica, Ringwood, New York 07456 (US); BARONE, Salvatore, Staten Island, New York 10314 (US)
(74) Vertreter: Reinstädler, Diane
(86) Internationale Anmeldenummer: PCT/EP2005/004495
(87) Internationale Veröffentlichungsnummer: WO 2005/102255

(56) Entgegenhaltungen:
- EP-A- 0 701 812
- EP-A- 1 258 290
- EP-A- 1 338 266
- WO-A-03/026608
- WO-A-03/105790
- DE-A1- 10 208 550
- US-A1- 2003 186 826
- US-A1- 2004 001 799

## Beschreibung

Die Erfindung betrifft ein insbesondere wasserhaltiges, schweißabsorbierendes kosmetisches Produkt, beispielsweise ein Deodorant, enthaltend eine Basisformulierung sowie einen schweißabsorbierenden Komplex. Die Erfindung betrifft ferner ein Verfahren zur Herstellung des Komplexes und des kosmetischen Produkts.

Kosmetische Produkte mit einer Antischweißwirkung sind bekannt. Es werden unterschiedliche Produktklassen unterschieden. Deodorants eliminieren unangenehme Gerüche, die von der bakteriellen Zersetzung von Schweiß entstehen. Deodorants enthalten daher häufig antibakterielle Substanzen sowie wohlriechende Substanzen, wie Duftstoffe. Viele ätherische Öle weisen sowohl antibakterielle als auch wohlriechende Eigenschaften auf, weswegen sie häufig in Deodorants Verwendung finden. Produkte, welche die Schweißproduktion an sich hemmen - so genannte Antiperspirantien - enthalten als Wirkstoff Adstringentien (Antiperspirantaktiva), insbesondere Aluminium- oder Zirkoniumsalze. Deodorantprodukte sind grundsätzlich wasserhaltig, wohingegen traditionelle Antiperspirantien (die in einer unpolaren Phase, wie Silikonöl oder Mineralöl, suspendierte Adstringentien enthalten) im Wesentlichen wasserfrei sind, um die Polymerisation der Adstringentien zu verhindern. Dennoch wurde unlängst ein neue Generation transparenter Antiperspirantien entwickelt, die aufgrund ihres ästhetischen Erscheinungsbildes an Popularität gewonnen hat. Diese neue Generation von Antiperspirantien sind Emulsionen, die üblicherweise Propylenglykol, Adstringentien, Wasser und Öle enthalten. Dieser Typus von Antiperspirantien ist jedoch wegen der Polymerisation der Adstringentien weniger aktiv als der traditionelle Typ.

Beide Produktklassen - Deodorants und Antiperspirantien - können zusätzlich Substanzen enthalten, die in der Lage sind, Schweiß zu absorbieren und so ein frisches und trockenes Hautgefühl zu vermitteln. Übliche schweißabsorbierende Komponenten sind beispielsweise Polymere, wie natürliche oder chemisch modifizierte Polysaccharide oder Polysaccharid-Gummis oder synthetische Polymere. Derartige Polymere bilden dreidimensionale Netzwerke aus, die in der Lage sind, Wasser aufzunehmen. Ein gemeinsames Problem von wasserhaltigen Produkten (umfassend Deodorants und Antiperspirantien vom Emulsionstyp) ist, dass die wasserabsorbierende Komponente schnell ihre Absorptionsfähigkeit verliert, sobald sie in eine wasserhaltige Formulierung eingebracht wird.

Aus WO 03/030853 A ist ein im Wesentlichen wasserfreies Unterarmprodukt bekannt, das ein spezielles wasserabsorbierendes Polymer, flüchtiges Silikon und ein Gelierungsmittel enthält und optional geringe Mengen eines Tensids (zur Stabilisierung der Formulierung), eines Antiperspirantwirkstoffs oder deodorierenden Mittels, nicht-flüchtigen Silikons und eines Emulgators. Das Produkt ist eine Suspension, in der das wasserabsorbierende Polymer in Form von dispergierten Partikeln vorliegt. Diese Formulierung ist nicht geeignet, um in übliche wasserhaltige Deodorantformulierungen eingesetzt zu werden, da das wasserabsorbierende Polymer in Wasser quillt und seine Wasserspeicherfähigkeit verliert.

Aufgabe der vorliegenden Erfindung ist, ein schweißabsorbierendes kosmetisches Produkt, insbesondere ein Deodorant, zur Verfügung zu stellen, das eine erhöhte und langanhaltende Wasserabsorptionsfähigkeit insbesondere in wasserhaltigen Formulierungen aufweist und die Formulierungsprobleme bekannter Deodorantprodukte überwindet.

Diese Aufgabe wird durch ein schweißabsorbierendes kosmetisches Produkt mit den Merkmalen des Anspruchs 1 gelöst. Der erfindungsgemäße Produkt enthält (in Massenanteilen):
i) eine Basisformulierung und
ii) einen schweißabsorbierenden Komplex, umfassend
   (a) 0,1 bis 90 % mindestens einer wasserabsorbierenden Komponente,
   (b) 0,1 bis 80 % mindestens eines oberflächenaktiven Agens,
   (c) 0,001 bis 20 %, insbesondere 0,01 bis 20 % mindestens eines Elektrolyts und
   (d) optional 0 bis 50 % mindestens eines Lösungsmittels und/oder mindestens einer Trägersubstanz.
   Dabei ist entscheidend, dass der schweißabsorbierende Komplex in der Basisformulierung in Form emulgierter Partikel vorliegt, welche aus einem dreidimensionalen, in Wasser quellbaren Polymernetzwerk der mindestens einen wasserabsorbierenden Komponente bestehen und welche mit dem mindestens einen oberflächenaktiven Agens zumindest teilweise umhüllt (beschichtet) sind.

In dem Komplex ist die mindestens eine wasserabsorbierende Komponente, die nicht vollständig in dem Lösungsmittel und/oder der Trägersubstanz quillt, aber in Wasser zu quellen vermag (um dabei Partikel eines dreidimensionalen gel-artigen polymeren Netzwerks auszubilden), aufgrund einer Beschichtung (Hülle) aus dem mindestens einen oberflächenaktiven Agens (Tensid) vor der Absorption von Wasser aus einer Basisformulierung des kosmetischen Produkts geschützt. Gleichzeitig stabilisiert die umhüllende Schicht des mindestens einen Tensids die Partikel des Absorptionsmaterials, wodurch seine Schweißabsorptionsfähigkeit konserviert wird. Die umhüllende Schicht aus dem oberflächenaktiven Agens sorgt zudem für eine homogene Suspendierung der Absorberpartikel in der Basisformulierung. Nach Auftragen auf die Haut verdampft/verflüchtigt das in der Basisformulierung enthaltene Wasser, wodurch das dehydratisierte Produkt einen dünnen Film auf der Haut bildet. Dieser Vorgang erlaubt dem Tensid, zwischen Haut und wasserabsorbierender Komponente zu verbleiben. Dabei unterstützt das oberflächenaktive Agens die Haftung der wasserabsorbierenden Komponente auf der Haut und vermittelt den Kontakt zwischen dieser und der Haut, der die Schweißaufnahme durch die Partikel ermöglicht. Aufgrund der Verkapselung quillt nur ein geringer Anteil der wasserabsorbierenden Komponente in dem Wasser der Formulierung, wodurch der größte Anteil der Wasserabsorptionsfähigkeit für die Schweißaufnahme zur Verfügung steht. Im Ergebnis bleibt die hohe Schweißabsorptionskapazität des Absorbermaterials über eine längere Dauer als bei bekannten Zusammensetzungen erhalten.

Das erfindungsgemäße kosmetische Produkt, insbesondere der schweißabsorbierende Komplex weist eine überragende Schweißabsorptionsfähigkeit, Feuchtigkeitsreduzierung, Inhibition von Mikroorganismen, und schließlich einen deutlichen deodorierenden Nutzen auf. Der Komplex ist geeignet, in jegliches kosmetische Produkt eingebracht zu werden, insbesondere in wasserhaltige Produkte, wie Unterarmdeodorants.

Nach besonders vorteilhaften Ausgestaltungen der Erfindung umfasst der schweißabsorbierende Komplex
(a) 10 bis 80 %, insbesondere vorzugsweise 20 bis 70 % und bevorzugt etwa 30 bis 50 % mindestens einer wasserabsorbierenden Komponente,
(b) 10 bis 70 %, insbesondere 20 bis 60 % und bevorzugt etwa 30 bis 45 % mindestens eines oberflächenaktiven Agens,
(c) 0,05 bis 15 %, insbesondere 0,1 bis 10 % und bevorzugt etwa 1 bis 5 % mindestens eines Elektrolyts und
(d) optional 0 bis 50 % mindestens eines Lösungsmittels und/oder mindestens einer Trägersubstanz.
Die Bereiche können abhängig von den Eigenschaften der einzelnen Komponenten sowie der Art des kosmetischen Produkts stark variieren.

Das mindestens eine Elektrolyt liegt vorzugsweise zumindest anteilsweise in den Zwischenräumen des Polymernetzwerks der mindestens einen wasserabsorbierenden Komponente inkorporiert vor, verursacht durch physikalische Kräfte, wie Wasserstoffbrückenbindungen und/oder hydrophobe Wechselwirkungen zwischen den Polymerketten. Diese Konformation verhindert übermäßig starke Wasserstoffbrückenbindungen der Backbone-Ketten der wasserabsorbierenden Komponente, so dass Wasser/Schweiß leicht eindringen und die Absorbermoleküle auseinander drängen kann, um diese rasch zu hydratisieren. Mit anderen Worten werden die inter- und intramolekularen Wasserstoffbrückenbindungen durch die Elektrolyte getrennt, um den Eintritt von Wasser zu erleichtern. Es wurde gezeigt, dass die Inkorporation von Elektrolyten in das Absorbermaterial die Schweiß- bzw. Wasserabsorptionskapazität des Polymernetzwerks noch weiter erhöht und das Wasser in den Zwischenräumen zurückhält. Auf diese Weise verstärken die Elektrolyte das erwünschte Trockengefühl auf der Haut.

Die mindestens eine wasserabsorbierende Komponente ist im Wesentlichen nicht in dem mindestens einen Lösungsmittel und/oder der Trägersubstanz löslich, andererseits aber in der Lage, in Wasser zu quellen, ohne vollständig in dem in einer Basisformulierung eines kosmetischen Produkts enthaltenen Wasser gelöst vorzuliegen. Sie weist eine Wasserabsorptionskapazität von mindestens 20 %, insbesondere mindestens 50 % bezogen auf ihre Trockenmasse auf. Die mittlere Partikelgröße der wasserabsorbierenden Komponente im Komplex liegt im Bereich von 0,1 bis 450 µm, insbesondere von 10 bis 200 µm. Partikel mit mittleren Partikelgrößen zwischen 50 bis 100 µm zeigen besonders gute Ergebnisse.

Das kosmetische Produkt, welches den oben beschriebenen schweißabsorbierenden Komplex enthält, kann aus einer vielfältigen Produktgruppe gewählt sein. Diese Produktgruppe umfasst Deodorants (insbesondere Unterarmdeodorants), jegliche deodorierende Präparationen, Stifte, Sprays, Aerosole, Cremes, Sonnenschutzmittel, Aftershaves, Lotionen, Grundierungscremes und Make-ups. Ein Vorteil des erfindungsgemäßen Komplexes ist, dass er ohne weiteres zu beliebigen Standardzubereitungen (Basisformulierungen) zugesetzt werden kann, insbesondere zu wasserhaltigen Formulierungen wie Deodorantbasen, ohne seine Wasserspeicherfähigkeit zu verlieren. Typischerweise enthalten solche Produkte-1 bis 40 Gew.-% Wasser, insbesondere 3 bis 20 %. Übliche Deodorantprodukte sind einphasige Formulierungen und enthalten etwa 12 Gew.-% Wasser.

Der schweißabsorbierende Komplex kann grundsätzlich in einem weiten Konzentrationsbereich von 0,05 bis 99 Gew.-% in kosmetischen Produkten eingesetzt werden. Üblicherweise ist der Komplex in einer Basisformulierung im Bereich von 0,1 bis 10 Gew.-%, vorzugsweise von 0,25 bis 5 % enthalten. In einer Deodorantbasisformulierung weist der Komplex vorteilhaft einen Massenanteil zwischen 0,1 und 10 %, insbesondere von etwa 1 % auf. Die Deodorantbasisformulierung selbst enthält üblicherweise 60 bis 90 Gew.-%, insbesondere 70 bis 80 % mindestens einen Lösungsmittels, 5 bis 20 %, insbesondere 10 bis 15 % Wasser und 1 bis 15 %, insbesondere 5 bis 8 % mindestens eines Gelierungsmittels.

Das kosmetische Produkt kann selbstverständlich weitere Hilfsstoffe und/oder aktive Agenzien enthalten, beispielsweise Pigmente, Farbstoffe, Antioxidantien, Konservierungsmittel, andere feuchtigkeitsspeichernde Substanzen, Weichmacher, Duftstoffe, Stabilisatoren, Zellturnover-Promotoren, Zellproliferationsstimulatoren, entzündungshemmende Agenzien, antimikrobielle Agenzien, Hormonregulatoren, Enzyminhibitoren, UV-Absorber, Sonnenschutzstoffe und dergleichen sowie Mischungen von diesen.

Wie bereits erwähnt, zeichnet sich der erfindungsgemäße kosmetische Produkt durch eine Beschichtung/Überzug der Absorberpartikel des schweißabsorbierenden Komplexes durch das mindestens eine Tensid aus. Dieses Gefüge des schweißabsorbierenden Komplexes wird durch ein Verfahren erhalten werden, das die Schritte umfasst:
(a) Mischen der mindestens einen wasserabsorbierenden Komponente und des mindestens einen Elektrolyts miteinander,
(b) Zugabe des mindestens einen oberflächenaktiven Agens unter Rühren und Wärmezufuhr, bis eine homogene Mischung erhalten wird,
   und - falls ein Lösungsmittel und/oder eine Trägersubstanz verwendet wird -
(c) Zugabe des mindestens einen Lösungsmittels und/oder der mindestens einen Trägersubstanz zu der Mischung aus Schritt (b) und Vermengen zu einer im Wesentlichen homogenen Mischung.

Die Zufuhr von thermischer Energie (Wärme) und Scherkräften (durch Rühren) in Schritt (b) ist entscheidend, um inter- und intramolekulare Bindungen innerhalb des oberflächenaktiven Agens und der wasserabsorbierenden Komponente aufzubrechen und im anschließenden Abkühlungsprozess die Bildung neuer physikalischer Wechselwirkungen (insbesondere hydrophobe Wechselwirkungen und Wasserstoffbrückenbindungen) zwischen dem zumindest einen Tensid und der zumindest einen wasserabsorbierenden Komponente zu ermöglichen. Ohne diese Zuführung von Wärme und Scherkräften bilden Tensid(e) und wasserabsorbierende Komponente(n) nur sehr geringe physikalische Wechselwirkungen aus und vermögen einem kosmetischen Produkt - nach Zusetzung zu einer kosmetischen Basisformulierung - keine signifikante Wasserabsorptionskapazität zu verleihen.

Um das kosmetische Produkt herzustellen, wird der so vorgefertigte schweißabsorbierende Komplex, der bereits die umhüllten Absorberpartikel enthält, einfach einer geeigneten Basisformulierung zugesetzt, insbesondere einer wasserhaltigen Deodorantbasisformulierung.

Nachfolgend werden geeignete Substanzen für die einzelnen Inhaltsstoffe des erfindungsgemäßen schweißabsorbierenden Komplexes genannt.

### Wasserabsorbierende Komponente

Die mindestens eine wasserabsorbierende Komponente wird ausgewählt aus beliebigen natürlichen oder synthetischen Polymeren, die in Wasser zu quellen vermögen und dabei ein dreidimensionales gel-artiges Netzwerk ausbilden.

Die vorliegend meist bevorzugten wasserabsorbierenden Komponenten stellen Gummis beziehungsweise gummiartige Polymere dar. Im Rahmen der vorliegenden Erfindung wird "Gummi" allgemein als ein beliebiges wasserlösliches Polymer definiert, das aus Land- oder Meerespflanzen oder -mikroorganismen isoliert werden kann und das die Fähigkeit besitzt, zur Viskosität und/oder Quellfähigkeit seiner Dispersion beizutragen. Die wasserabsorbierende Komponente ist vorzugsweise ein aus pflanzlicher oder mikrobieller Biosynthese, von Land oder Meeresorganismen stammender Gummi. Solche Gummis enthalten im Wesentlichen Wiederholungen von Monosaccharideinheiten und weisen relativ hohe Molmassen von vorzugsweise mindestens 100.000 g/mol auf. Beispiele bevorzugter Gummis sind Guar-Gummi (Cyamopsis tetragonolobus-Gummi), Guar-Derivate, Johannisbrotkernmehl, Cellulose-Gummi, Gummi arabicum, Karaya-Gummi, Traganth, aus Algen stammende Gummis (umfassend Agar, Carrageen, Furcellaran und Alginate), Scleroglucan (Sclerotium gum), Tamarindenkernmehl, Xanthan, Dextran, Gellan, Propylenglycolalginat und dergleichen sowie Derivate und Mischungen von diesen.

Eine weitere bevorzugte Klasse wasserabsorbierender Komponenten stellen Polysaccharide pflanzlichen Ursprungs dar, die im Wesentlichen eine Wiederholung von Monosaccharideinheiten von Hexosen und/oder Pentosen umfassen. Besondere Beispiele umfassen natürliche Cellulosen, natürliche Fasern, Cellulose-Derivate, mikrokristalline Cellulose, Carboxymethylcellulosen, Methylcellulosen, Hydroxyethylcellulosen, Hydroxypropylcellulosen, Hydroxypropylmethylcellulosen, Pektine, Maltodextrine, Inulin, Inulin-Derivate, Stärke, Stärke-Derivate, Derivate und Mischungen von diesen.

Noch eine andere Gruppe geeigneter wasserabsorbierender Komponenten wird aus chemisch modifizierten Polysacchariden gewählt, insbesondere aus der Gruppe enthaltend Cellulose-Derivate, Stärke-Derivate, Pektin-Derivate, Stärke/Acrylamid/Natriumacrylat-Pfropfcopolymere (Stärkepfropfpolymere) und Mischungen von diesen.

Gemäß einer weiteren Ausgestaltung der Erfindung wird die mindestens eine wasserabsorbierende Komponente aus Polyacrylat-basierten synthetischen Polymeren gewählt, die insbesondere Wiederholungen von Acrylsäure, Acrylamid, Methacrylsäure, Derivaten oder Mischungen von diesen umfassen. Beispiele dieser Verbindungen umfassen Natriumpolyacrylate, Polyacrylamide, ihre Copolymere und Mischungen von diesen.

Eine andere Gruppe bevorzugter wasserabsorbierender Komponenten ist gewählt aus Kieselsäuren (Si(OH)₄ bzw. SiO₂) und beliebigen Arten ihrer Derivate und Modifikationen. Geeignete Beispiele umfassen ihre Kondensationsprodukte Polykieselsäuren ((SiO₂)ₘ x nH₂O), Kieselsäureanhydrid (Silica, SiO₂), pyrogene Kieselsäure ("Fumed Silica"), hydratisierte Kieselsäure (SiO₂ x nH₂O), Silicagel, Silikatester und/oder Silikatsalze, wie Natriumsilikat, Magnesiumsilikat und Calciumsilikat. Es wurde gefunden, dass die Wasserabsorptionsrate und -kapazität dieser Kieselsäurenkomponenten dramatisch verstärkt werden kann, indem sie vor der Herstellung des Komplexes in einer Ölphase zunächst vordispergiert werden.

Selbstverständlich können Mischungen der vorstehenden wasserabsorbierenden Komponenten ebenfalls eingesetzt werden.

### Oberflächenaktive Agenzien

Wie bereits ausgeführt, hat das mindestens eine oberflächenaktive Agens die Funktion, eine Beschichtung auf der äußeren Oberfläche der Partikel der wasserabsorbierenden Komponente(n) auszubilden, wodurch ihre Oberflächeneigenschaften beeinflusst werden. Insbesondere helfen die oberflächenaktiven Agenzien, das Polymer auf der Haut zu halten, und bewahren es vor unerwünschter Wasserabsorption aus der Basisformulierung. Oberflächenaktive Agenzien umfassen Verbindungen - enthaltend Monomere, Dimere, Trimere, Oligomere und Polymere -, die lipophile genauso wie hydrophile Funktionalitäten ausreichender Stärke aufweisen, um Affinitäten sowohl gegenüber hydrophilen als auch gegenüber lipophilen Anteilen der Formulierung auszubilden. Solche Agenzien bilden orientierte Schichten um die wasserabsorbierenden Partikel aus. Auf diese Weise werden die Partikel der wasserabsorbierenden Komponente(n) innerhalb der Formulierung stabilisiert und homogen verteilt.

Für die oben genannten Zwecke weist das oberflächenaktive Agens eine so genannte Hydrophilie/Lipophilie-Balance (HLB) unterhalb von 13, insbesondere im Bereich von 2 bis 13, vorzugsweise von 3 bis 11 auf. Sehr gute Resultate werden mit Tensiden mit einem HLB im Bereich von 3 bis 9, bevorzugt von 4 bis 8 erhalten. Der HLB ist ein von C. Griffin entwickelter dimensionsloser Wert, der die relativen Anteile der lipophilen gegenüber der hydrophilen Segmente eines Materials berücksichtigt. Die Zuweisung von numerischen Werten für den HLB basiert auf Effekten der chemischen Gruppen innerhalb des Moleküls (D.L. Courtney, in "Surfactants in Cosmetics", 2. Auflage, Marcel Dekker Inc., New York, 1997, 128-130).

Gemäß einer bevorzugten Ausgestaltung der Erfindung wird eine Mischung von mindestens zwei oberflächenaktiven Agenzien in dem Komplex verwendet. In diesem Fall können Tenside mit HLB-Werten auch außerhalb der oben angegebenen Bereiche eingesetzt werden, vorausgesetzt, dass ein effektiver gewichteter mittlerer HLB der Kombination in die oben genannten Bereiche fällt.

Das mindestens eine oberflächenaktive Agens kann eine nicht-ionische, anionische, kationische oder amphotere Verbindung oder eine Kombination von solchen sein. Ausgewählte Beispiele enthalten Fettalkohole, ethoxylierte Alkohole, ethoxylierte Triglyceride, ethoxylierte Öle, Monoglyceride, Carboxylsäureester von Alkyl- oder Alkenylglycolen, C1-C40-Fettsäureester von Polyolen, C1-C40-Fettsäureether von Polyolen, C1-C40- Fettsäureester von Alkyl- oder Alkenylglycolen, Polyglycerinester, Polyglycerinester von C1-C40-Fettsäuren, Kohlenwasserstoff-abgeleitete Ester, Zuckerester und Polyester, alkoxylierte Zuckerester und Polyester, ethoxylierte Carboxylsäureester von C1-C40-Fettsäuren, Sorbitan- oder Polysorbatester von Fettsäuren, ethoxylierte Sorbitanester von Fettsäuren, ethoxylierte Zuckerester von Fettsäuren, alkoxylierte Derivate von C1-C40-Fettsäureestern von C1-C40-Fettalkoholen, alkoxylierte Derivate von C1-C40-Fettsäureethern von C1-C40-Fettalkoholen, Polyethylenglycolether, Polyethylenglycolester, ethoxylierte Polysiloxane, Alkylglycoside, Alkanolamide, Aminoxide, Cetylphosphat, Kaliumcetylphosphat, Diethanolamincetylphosphat, Carboxylsäuren und deren Derivate, Sulfonsäurederivate, Schwefelsäurederivate, Phosphorsäurederivate, ethoxylierte Fettetherphosphate, Fettsäureamide, Acyllactylate, Alkylamidoalkylamine, Alkylamine, Alkylimidazoline, alkylsubstituierte Aminosäuren und Mischungen von diesen. Besondere Beispiele sind Saccharosestearat und Sorbitan sesquioleat und Mischungen von diesen.

### Elektrolyte

Aufgabe des mindestens einen Elektrolyts ist, den Komplex (insbesondere die Partikel) zu stabilisieren und die Wasserabsorption durch elektrostatische Abstoßungswechselwirkungen zwischen den Ionen, Kräften zwischen den Polymerketten und den osmotischen Druck der Partikel zu erhöhen. Auf diese Weise halten die Elektrolyte das Wasser innerhalb der Partikel zurück und helfen die Stabilität und Struktur der Partikel zu aufrechtzuerhalten.

Geeignete Elektrolyten können eine oder mehrere Arten sein, ausgewählt aus der Gruppe enthaltend Alkalimetallsalze, Ammoniak, niedermolekulare Amine, Salze von di- oder trivalenten Kationen, Phosphatsalze, Steinextrakte und Mischungen von diesen. Besondere Beispiele sind Natriumcitrat, Natriumchlorid, Kaliumcitrat, Natriumhexametaphosphat, Calciumchlorid. Calciumcarbonat und Mischungen von diesen.

Die notwendigen Massenanteile des Elektrolyts im wasserabsorbierenden Komplex hängen von den physikalischen und chemischen Eigenschaften sowohl des Elektrolyts als auch der übrigen Komponenten ab.

### Lösungsmittel/Trägersubstanz

Die Gegenwart eines Lösungsmittels und/oder einer Trägersubstanz im schweißabsorbierenden Komplex ist optional. Sie ist in der Regel nur dann notwendig, wenn das mindestens eine oberflächenaktive Agens ein Feststoff ist. Im Falle eines oberflächenaktiven Agens, das als Flüssigkeit vorliegt, besteht keine Notwendigkeit für ein Lösungsmittel und/oder eine Trägersubstanz.

Das mindestens eine Lösungsmittel und/oder die mindestens eine Trägersubstanz kann ausgewählt werden aus der Gruppe enthaltend Glycole, Glycerin, polare und unpolare Öle, Kohlenwasserstoffe, Ether, Ester, mittel- und langkettige Alkohole, alkoxylierte Alkohole, polyhydrierte Alkohole, Polyole und Mischungen von diesen. Besondere Beispiele umfassen Propylenglycol, Dipropylenglycol, Ethylenglycol, Glycerin, Diglycerin, Diacetin, Triacetin, Isopropylpalmitat, Isododecan, Isohexadecan, Triglyceride, Mineralöl und Mischungen von diesen.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figur 1: zeitlicher Verlauf der Wasserabsorption verschiedener unverarbeiteter wasserabsorbierender Komponenten;
- Figur 2: Wasserspeicherkapazität von unverarbeiteten wasserabsorbierenden Komponenten in einer Deodorantbasisformulierung;
- Figur 3: Wasserspeicherkapazität von schweißabsorbierenden Komplexen in einer Deodorantbasisformulierung gemäß einer ersten Ausgestaltung der Erfindung und
- Figur 4: Wasserspeicherkapazität von schweißabsorbierenden Komplexen in einer Deodorantbasisformulierung gemäß einer weiteren Ausgestaltung der Erfindung.

### (1) Herstellung des schweißabsorbierenden Komplexes

Mindestens eine wasserabsorbierende Komponente wird in einen sauberen, trockenen Edelstahlkessel gegeben, der mit einem Rührer ausgestattet ist. Dann wird mindestens ein Elektrolyt zugegeben. Unter langsamen Rühren wird das Gemenge auf eine Temperatur zwischen 50 und 100 °C aufgeheizt und bei dieser Temperatur gehalten. Anschließend wird mindestens ein oberflächenaktives Agens langsam in den Kessel zugegeben. Unter Beibehaltung der Batch-Temperatur'von 50-100 °C wird das Gemisch für mindestens weitere 15 Minuten kontinuierlich gerührt, bis ein im Wesentlichen einheitliches (homogenes) Gemisch erhalten wird, das keine ungelösten Rohmaterialien enthält. Abhängig von den einzelnen Inhaltsstoffen hat der so hergestellte Komplex die Konsistenz einer Paste, eines weichen Feststoffes oder eines harten Wachses.

Die genauen Mengen der Inhaltsstoffe hängen von den ausgewählten Substanzen ab. Typische massenbezogene Verhältnisse sind: wasserabsorbierende Komponente(n) : Elektrolyt(e) : oberflächenaktive(s) Agens/Agenzien : 50 : 5 : 45. Das Massenverhältnis der wasserabsorbierenden Komponente(n) zu den oberflächenaktive Agens/Agenzien beträgt 1 : (0,25-2), vorzugsweise 1 : (0,5-1,5). Die bevorzugte Batch-Temperatur für das Verfahren hängt von den Schmelzpunkten der Inhaltsstoffe, insbesondere der wasserabsorbierenden Komponente und des oberflächenaktive Agens ab. Es variiert zwischen 50 und 100 °C, insbesondere zwischen 60 und 90 °C. Für die meisten Inhaltsstoffe ist eine Batch-Temperatur zwischen 70 und 80 °C geeignet.

Im Falle der Verwendung eines festen oberflächenaktiven Agens wird anschließend noch ein Lösungsmittel und/oder eine Trägersubstanz der Mischung aus wasserabsorbierender Komponente, Elektrolyt und oberflächenaktivem Agens zugesetzt und so lange gerührt, bis eine homogene Mischung ohne ungelöste Rohmaterialien vorliegt.

### (2) Herstellung eines Deodorantstifts

0,1-10 g, insbesondere 1-2 g eines nach (1) hergestellten schweißabsorbierenden Komplexes wird einer geschmolzenen Deodorantbasisformulierung unter ständigem Rühren bei einer Batch-Temperatur von 60-90 °C, insbesondere von 70-80 °C, zugesetzt, so dass eine homogene Mischung von insgesamt 100 g erhalten wird. Die Deodorantbasisformulierung enthält üblicherweise 60-90 %, insbesondere 65-80 Gew.-% mindestens eines Lösungsmittels (beispielsweise Propylenglycol); 5-30 %, insbesondere 10-25 % Wasser und 1-15 %, insbesondere 5-13 % mindestens eines Gelierungsmittels (beispielsweise Natriumstearat). Die Mischung wird anschließend auf Raumtemperatur heruntergekühlt, um eine Stiftform zu erhalten.

### (3) Wasserabsorptionsverhalten verschiedener wasserabsorbierender Komponenten (unverarbeitete Rohstoffe)

Eine definierte Menge jedes Rohstoffs (unverarbeitete wasserabsorbierende Komponente) wurde in eine Feuchtigkeitskammer mit einer relativen Luftfeuchte von 95-99 % gegeben und dort für acht Wochen inkubiert. Die prozentuale Wasserabsorption jeder Probe wurde durch Differenzwägung der Probe vor und nach der Inkubation in der Feuchtigkeitskammer und durch Division des Massenunterschiedes durch die Probenmasse vor der Inkubation berechnet.

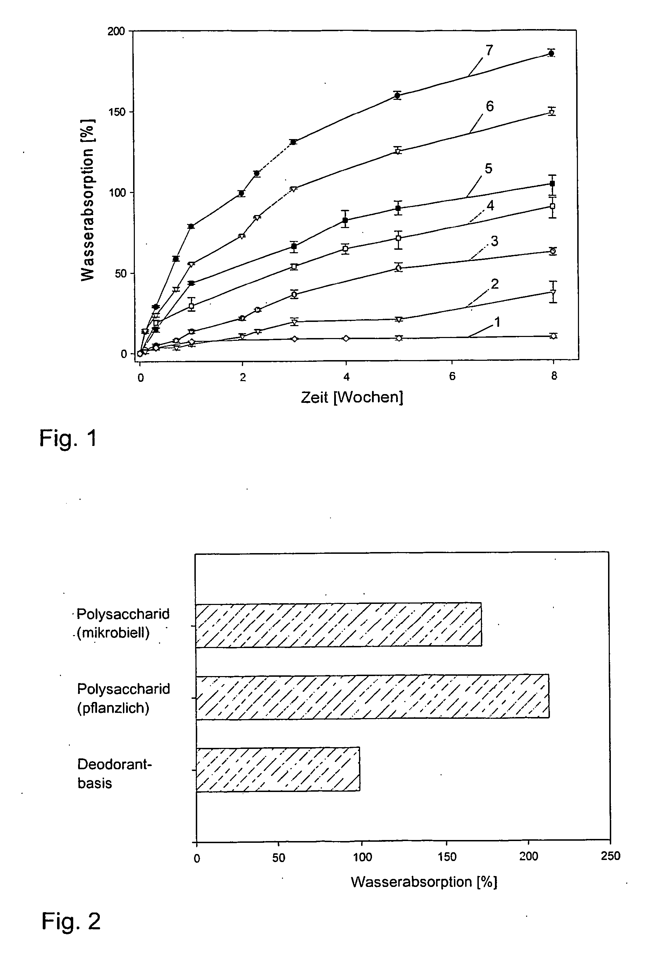

Figur 1 zeigt den achtwöchigen Verlauf der Wasserabsorption einiger Beispiele von potentiellen Kandidaten für die wasserabsorbierende Komponente. Die Ergebnisse folgender wasserabsorbierenden Komponenten sind dargestellt: Mikrokristalline Cellulose (Kurve 1), Kieselsäureanhydrid (Silica) (2), Cellulosepulver (3), Polysaccharid-Gummi (4), Cellulose-Gummi (5), Stärkepfropfpolymer (6) und Salz einer Polyacrylsäure = Natriumpolyacrylat (7). Die Daten zeigen deutlich, dass Polysaccharid-Gummi, Cellulose-Gummi, Stärkepfropfpolymer und Natriumpolyacrylat (Kurven 4-7) jeweils eine Wasserspeicherkapazität von über 100 % bezogen auf die Trockenmasse des Rohmaterials aufweisen. Diese Komponenten werden daher bevorzugt in den erfindungsgemäßen schweißabsorbierenden Komplexen eingesetzt.

### (4) Wasserabsorptionsverhalten verschiedener wasserabsorbierender Komponenten in einer Deodorantbasisformulierung

Jeweils 5 g der unverarbeiteten wasserabsorbierenden Komponente (Rohmaterial) wurden mit 95 g geschmolzener Deodorantbasisformulierung (70-80 Gew.-% Propylenglycol, 10-15 % H₂O, 5-8 % Natriumstearat) verrührt, bis ein homogenes Gemisch erhalten wurde. Die Mischung wurde auf Raumtemperatur gekühlt, um eine Stiftform zu erhalten.
Jeweils eine definierte Menge dieser Mischungen wurde auf Glaswolle in einen geschlossenen Behälter mit Wasserüberschuss platziert und anschließend bei 37 °C für 24 Stunden inkubiert. Eine Probe der reinen Deodorantbasisformulierung wurde auf die gleiche Weise in dem Behälter inkubiert. Nachdem der Überschuss an Wasser vorsichtig aus dem Behälter dekantiert wurde, wurde die prozentuale Wasserabsorption durch Differenzwägung jeder Probe vor und nach der Wasseraussetzung bestimmt.
Die Ergebnisse für ein Polysaccharid-Gummi aus mikrobieller Biosynthese (Sclerotium gum von Alban Muller Ind.) und ein Polysaccharid-Gummi pflanzlichen Ursprungs (Blend aus Xanthan und Cyamopsis tetragonolobus-Gummi (=Guar) von TIC gums, INC.) sind in Figur 2 dargestellt. Zudem ist die Wasserabsorption der puren Deodorantbasisformulierung als Referenz gezeigt. Die Ergebnisse sind in g absorbierten Wassers pro g der Deodorantbasis mit bzw. ohne Polysaccharid vor Inkubation angegeben. Die Daten zeigen, dass die Polysaccharid-Gummis-enthaltenden Deodorants mehr als das 1,5-Fache an Wasser absorbieren als das reguläre Deodorant ohne Polysaccharidzusatz. Das belegt, dass der Zusatz von 1 Gew.-% an Polysaccharid-Gummi einer regulären, wasserhaltigen Deodorantbasisformulierung eine zusätzliche Wasserspeicherkapazität von 15 bis 23 % verleiht.

### (5) Wasserabsorptionsverhalten verschiedener schweißabsorbierender Komplexe in einer Deodorantbasisformulierung nach einer ersten erfindungsgemäßen Ausgestaltung

Ein elektrolythaltiger schweißabsorbierender Komplex gemäß der vorliegenden Erfindung (Komplex A) sowie ein elektrolytfreier Komplex (Komplex B) wurden nach der oben beschriebenen Prozedur hergestellt. Die Zusammensetzungen der beiden Komplexe sind in Tabelle 1 angegeben.

Jeweils 1 g der Komplexe wurde mit 97,5 g einer geschmolzenen Deodorantbasisformulierung (70-80 Gew.-% Propylenglycol, 10-15 % H₂O, 5-8 % Natriumstearat) und mit 1,5 g eines Duftstoffes verrührt, bis ein homogenes Gemisch erhalten wurde. Das Gemisch wurde auf Raumtemperatur gekühlt, um eine Stiftform zu erhalten. Die so erhaltenen Produkte enthielten somit 1 Gew.-% des jeweiligen Komplexes bzw. 0,5 Gew.-% der wasserabsorbierenden Komponente.

Jeweils eine definierte Menge dieser Mischungen wurde auf Glaswolle in einen geschlossenen Behälter mit Wasserüberschuss platziert und anschließend bei 37 °C für 24 Stunden inkubiert. Nachdem der Überschuss an Wasser vorsichtig aus dem Behälter dekantiert wurde, wurde die prozentuale Wasserabsorption durch Differenzwägung jeder Probe vor und nach der Wasseraussetzung bestimmt.

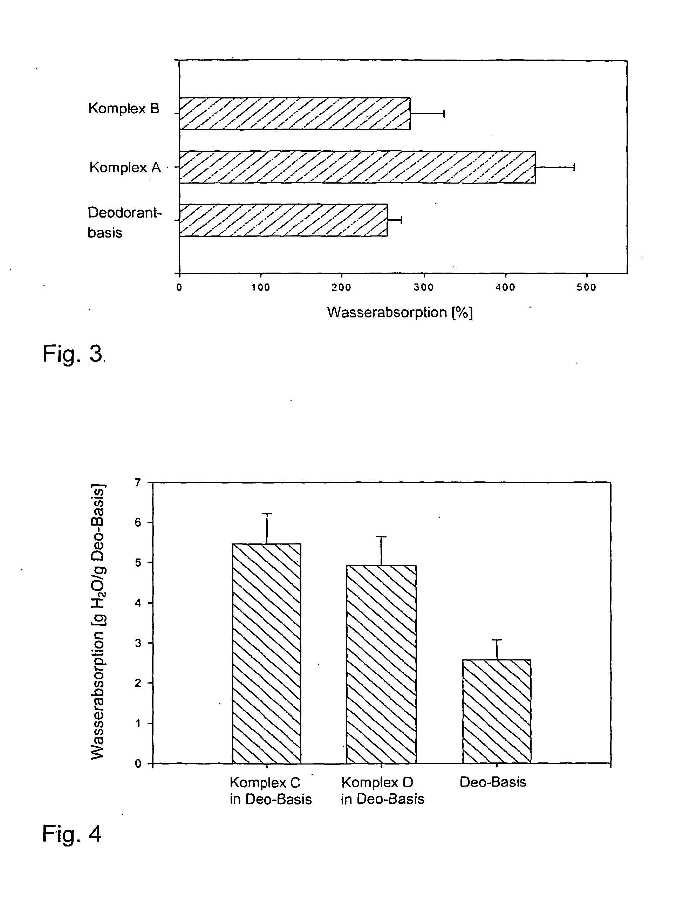

Die Ergebnisse für Komplex A und B sowie für die pure, den Duftstoff enthaltende Deodorantbasisformulierung sind in Figur 3 dargestellt. Die gegenüber der Deodorantbasisformulierung in Figur 2 erhöhte Wasserspeicherkapazität der reinen Deodorantbasisformulierung ist auf den Duftstoff zurückzuführen, der einen positiven Einfluss auf die Wasserabsorption hat. Die Daten zeigen, dass die Deodorants mit den Komplexen A und B mehr als das 0,7-beziehungsweise 0,2-Fache an Wasser gegenüber dem regulären Deodorant absorbieren können. Darüber hinaus belegen die Daten, dass die Elektrolyte einen sehr positiven Einfluss auf die Wasserspeicherkapazität des Absorbermaterials haben. Im Ergebnis führt 1 Gew.-% eines in Form des erfindungsgemäßen Komplexes zugesetzten Polysaccharid-Gummis zu einer zusätzlichen Wasserspeicherkapazität von 360 % gegenüber der regulären Deodorantbasisformulierung. Das Beispiel belegt, dass der erfindungsgemäße schweißabsorbierende Komplex gegenüber der puren wasserabsorbierenden Komponente im Deodorant zu einer wesentlich höheren Wasserspeicherkapazität führt.

**Tabelle 1:**

| | Komplex A | Komplex B |
|---|---|---|
| Polysaccharid-Gummi (Xanthan + Cyamopsis Tetragonoloba-Gummi) | 50% | 50% |
| oberflächenaktives Agens, berechneter HLB = 7,5 (Saccharosestearat + Sorbitan-Sesquioleat) | 45% | 50% |
| Elektrolyt (Natriumcitrat) | 5% | - |
| Summe | 100 % | 100% |

### (6) Wasserabsorptionsverhalten verschiedener schweißabsorbierender Komplexe in einer Deodorantbasisformulierung nach einer zweiten erfindungsgemäßen Ausgestaltung

Analog zu Ausführungsbeispiel 5 wurden zwei Typen wasserabsorbierender Komplexe (Komplex C und D) mit den in Tabelle 2 angegebenen Zusammensetzungen (in g oder Gew.-%) hergestellt. Die Komplexe unterscheiden sich im Wesentlichen in den Anteilen des Elektrolyts (Natriumcitrat). Diese Komplexe wurden - wie oben beschrieben - in eine Deodorant-Basisformulierung eingearbeitet, um Deodorantprodukte mit den in Tabelle 3 angegebenen Zusammensetzungen (in g oder Gew.-%) zu erhalten. Dabei kennzeichnen die in Tabelle 2 und 3 genannten Bereichsangaben ungefähr die in den Testreihen abgedeckten Bereiche. Die in den Klammern angegebenen Werte entsprechen hingegen konkreten Beispielen. Im Ergebnis enthielt das Produkt enthaltend Komplex C (Test 76-1) 0,05 Gew.-% Elektrolyt und das Produkt enthaltend Komplex D, (Test 76-2) 0,001 Gew.-% Elektrolyt. Ein Vergleichsbeispiel ohne Komplex und damit ohne Elektrolyt wurde ebenfalls hergestellt (Test 76-3).

Die Proben wurden in einem geschlossenen Behälter auf Glaswolle gewogen. Nach Zugabe von Wasser in den Behälter wurden die Proben bei 37 °C für 24 Stunden inkubiert. Nach vorsichtigem Dekantieren des Wassers wurden die Proben erneut ausgewogen und die Wasserabsorption durch Differenzwägung vor und nach der Wasseraussetzung bestimmt. Das in Figur 4 dargestellte Ergebnis zeigt zwar eine etwas höhere Wasserabsorption der elektrolytreichen Probe Test 76-1 (Komplex C) gegenüber der weniger elektrolythaltigen Probe Test 76-2 (Komplex D). Jedoch ist dieser Unterschied wenig signifikant (gemäß "Student's T-Test", p=0,28 und n=5). Hingegen ist die Wasserabsorption der Komplexhaltigen Proben Test 76-1 und 76-2 gegenüber der Komplex-freien Probe Test 76-3 (reine

**Tabelle 2:**

| | **Komplex C** | **Komplex D** |
|---|---|---|
| Propylenglycol | 30-35 (33,4) | 33-40 (36,6) |
| Natriumcitrat | 3-5 (3,3) | 0,05-0,1 (0,07) |
| Natur-Baumwolle | 0,1-0,5 (0,2) | 0,1-0,5 (0,2) |
| Xanthan + Guar-Gummi | 30-35 (33,1) | 30-35 (33,1) |
| Saccharosestearat | 8-15 (10) | 8-15 (10) |
| Sorbitan-Sesquioleat | 18-23 (20) | 18-23 (20) |
| **Summe** | 100 | 100 |

**Tabelle 3:**

| | **Test 76-1 Komplex C in Deo-Basis** | **Test 76-2 Komplex D in Deo-Basis** | **Test 76-3 Deo-Basis** |
|---|---|---|---|
| Propylenglycol | 65-70 (67,8) | 65-70 (67,8) | 67-72 (69,3) |
| Wasser, deionisiert | 17-23 (19) | 17-23 (19) | 17-23(19) |
| Triclosan | 0,1-0,5 (0.3) | 0,1-05 (0,3) | 0,1-0,5 (0,3) |
| Natriumstearat | 7-12 (9) | 7-12 (9) | 7-12 (9) |
| Stearinsäure | 0,5-1 (0,75) | 0,5-1 (0,75) | 0,5-1 (0,75) |
| Wasserabsorb. Komplex C (Tab. 2) | 1-2 (1.5) | - | - |
| Wasserabsorb. Komplex D (Tab. 2) | - | 1-2 (1,5) | - |
| Duftstoff | n.B. | n.B. | n.B. |
| Allantoin | 0,1-1 (0,1) | 0,1-1 (0,1) | 0,1-1 (0,1) |
| **Summe** | 100 | 100 | 100 |

| | | | |
|---|---|---|---|
| n.B. = nach Bedarf, abhängig vom Duftstoff | | | |

## Patentansprüche

1. Schweißabsorbierendes kosmetisches Produkt enthaltend
i) eine Basisformulierung und
ii) einen schweißabsorbierenden Komplex, umfassend
(a) 0,1 bis 90 Gew.-% mindestens einer wasserabsorbierenden Komponente,
(b) 0,1 bis 80 Gew.-% mindestens eines oberflächenaktiven Agens,
(c) 0,001 bis 20 Gew.-% mindestens eines Elektrolyten und
(d) 0 bis 50 Gew.-% mindestens eines Lösungsmittels und/oder mindestens einer Trägersubstanz,
worin der schweißabsorbierende Komplex Partikel aus einem dreidimensionalen, in Wasser quellbaren Netzwerk der mindestens einen wasserabsorbierenden Komponente ausbildet, welche mit dem mindestens einen oberflächenaktiven Agens zumindest teilweise umhüllt sind und in der Basisformulierung emulgiert sind,
wobei das kosmetische Produkt nach einem Verfahren herstellbar ist, das die Schritte umfasst:
(I) Mischen der mindestens einen wasserabsorbierenden Komponente und des mindestens einen Elektrolyten,
(II) Zugabe des mindestens einen oberflächenaktiven Agens unter Rühren und Wärmezufuhr, bis eine im Wesentlichen homogene Mischung erhalten wird,
(III) sofern im Komplex enthalten, Zugabe des mindestens einen Lösungsmittels und/oder der mindestens einen Trägersubstanz unter Rühren, bis eine im Wesentlichen homogene Mischung erhalten wird, und
(IV) Mischen des so erhaltenen schweißabsorbierenden Komplexes mit der Basisformulierung.

2. Kosmetisches Produkt nach Anspruch 1, **dadurch gekennzeichnet, dass** der mindestens eine Elektrolyt einen Massenanteil im schweißabsorbierenden Komplex von 0,01 bis 20 Gew.-% aufweist.

3. Kosmetisches Produkt nach Anspruch 1 oder 2, **dadurch gekennzeichnet, dass** der schweißabsorbierende Komplex umfasst:
(a) 10 bis 80 Gew.-%, insbesondere 20 bis 70 Gew.-%, der mindestens einen wasserabsorbierenden Komponente,
(b) 10 bis 70 Gew.-%, insbesondere 20 bis 60 Gew.-%, des mindestens einen oberflächenaktiven Agens,
(c) 0,05 bis 15 Gew.-%, insbesondere 0,1 bis 10 Gew.-% des mindestens einen Elektrolyten und
(d) 0 bis 50 Gew.-% des mindestens einen Lösungsmittels und/oder der mindestens einen Trägersubstanz.

4. Kosmetisches Produkt nach einem der vorhergehenden Ansprüche, **dadurch gekennzeichnet, dass** zumindest Anteile des mindestens einen Elektrolyten in Zwischenräumen des dreidimensionalen Netzwerks der mindestens einen wasserabsorbierenden Komponente inkorporiert sind.

5. Kosmetisches Produkt nach einem der vorhergehenden Ansprüche, **dadurch gekennzeichnet, dass** die mindestens eine wasserabsorbierende Komponente in Kontakt mit Wasser Partikel eines dreidimensionalen polymeren Netzwerks ausbildet.

6. Kosmetisches Produkt nach Anspruch 5, **dadurch gekennzeichnet, dass** die mindestens eine wasserabsorbierende Komponente Partikel mit mittleren Partikelgrößen im Bereich von 0,1 bis 450 µm, insbesondere von 10 bis 200 µm, vorzugsweise von 50 bis 100 µm ausbildet.

7. Kosmetisches Produkt nach einem der vorhergehenden Ansprüche, **dadurch gekennzeichnet, dass** die mindestens eine wasserabsorbierende Komponente eine Wasserabsorptionskapazität von mindestens 20 %, insbesondere mindestens 50 % bezogen auf ihre Trockenmasse aufweist.

8. Kosmetisches Produkt nach einem der vorhergehenden Ansprüche, **dadurch gekennzeichnet, dass** die mindestens eine wasserabsorbierende Komponente in dem mindestens einen Lösungsmittel und/oder der Trägersubstanz im Wesentlichen unlöslich ist.

9. Kosmetisches Produkt nach einem der vorhergehenden Ansprüche, **dadurch gekennzeichnet, dass** die mindestens eine wasserabsorbierende Komponente aus Gummis aus pflanzlicher oder mikrobieller Biosynthese gewählt ist, die im Wesentlichen eine Wiederholung von Monosaccharideinheiten umfassen und eine molekulare Masse von mindestens 100.000 g/mol aufweisen, insbesondere aus der Gruppe gewählt ist enthaltend Guar-Gummi, Guar-Derivate, Johannisbrotkernmehl, Cellulose-Gummi, Gummi arabicum, Karaya-Gummi, Traganth, Algen-Gummis (umfassend Agar, Carrageen, Furcellaran und Alginate), Scleroglucan, Tamarindenkernmehl, Xanthan, Dextran, Gellan, Propylenglycolalginat, Derivate und Mischungen von diesen.

10. Kosmetisches Produkt nach einem der vorhergehenden Ansprüche, **dadurch gekennzeichnet, dass** die mindestens eine wasserabsorbierende Komponente aus Polysacchariden pflanzlichen Ursprungs gewählt ist, die im Wesentlichen eine Wiederholung von Monosaccharideinheiten von Hexosen und/oder Pentosen umfassen, insbesondere aus der Gruppe gewählt ist enthaltend natürliche Cellulosen, natürliche Fasern, Cellulose-Derivate, mikrokristalline Cellulose, Carboxymethylcellulosen, Methylcellulosen, Hydroxyethylcellulosen, Hydroxypropylcellulosen, Hydroxypropylmethylcellulosen, Pektine, Maltodextrine, Inulin, Inulin-Derivate, Stärke, Stärke-Derivate, Derivate und Mischungen von diesen.

11. Kosmetisches Produkt nach einem der vorhergehenden Ansprüche, **dadurch gekennzeichnet, dass** die mindestens eine wasserabsorbierende Komponente aus chemisch modifizierten Polysacchariden gewählt ist, insbesondere aus der Gruppe enthaltend Cellulose-Derivate, Stärke-Derivate, Pektin-Derivate, Stärke/Acrylamid/Natriumacrylat-Pfropfcopolymere (Stärkepfropfcopolymere) und Mischungen von diesen.

12. Kosmetisches Produkt nach einem der vorhergehenden Ansprüche, **dadurch gekennzeichnet, dass** die mindestens eine wasserabsorbierende Komponente aus auf Polyacrylaten basierenden synthetischen Polymeren gewählt ist, die insbesondere Wiederholungen von Acrylsäure, Acrylamid, Methacrylsäure, Derivate oder Mischungen von diesen umfassen, insbesondere aus der Gruppe gewählt sind enthaltend Natriumpolyacrylate, Polyacrylamide, ihre Copolymere und Mischungen von diesen.

13. Kosmetisches Produkt nach einem der vorhergehenden Ansprüche, **dadurch gekennzeichnet, dass** die mindestens eine wasserabsorbierende Komponente aus Kieselsäuren und ihren Derivaten und Modifikationen gewählt ist, insbesondere aus der Gruppe enthaltend Polykieselsäuren ((SiO₂)ₘ x nH₂O), Kieselsäureanhydrid (SiO₂), pyrogene Kieselsäure, hydratisierte Kieselsäure (SiO₂ x H₂O), Silicagel, Silikatsalze und/oder Silikatester.

14. Kosmetisches Produkt nach einem der vorhergehenden Ansprüche, **dadurch gekennzeichnet, dass** das mindestens eine oberflächenaktive Agens eine Hydrophilie/Lipophilie-Balance (HLB) im Bereich von 2 bis 13, insbesondere von 3 bis 11, speziell von 3 bis 9, bevorzugt von 4 bis 8, aufweist.

15. Kosmetisches Produkt nach einem der vorhergehenden Ansprüche, **dadurch gekennzeichnet, dass** der Komplex eine Mischung oberflächenaktiver Agenzien aufweist, die eine effektive gewichtete mittlere Hydrophilie/Lipophilie-Balance (HLB) im Bereich von 2 bis 13, insbesondere von 3 bis 11, speziell von 3 bis 9, bevorzugt von 4 bis 8, aufweist.

16. Kosmetisches Produkt nach einem der vorhergehenden Ansprüche, **dadurch gekennzeichnet, dass** das mindestens eine oberflächenaktive Agens eine nicht-ionische, anionische, kationische oder amphotere Verbindung oder ein Mischung von diesen ist, insbesondere gewählt ist aus der Gruppe enthaltend Fettalkohole, ethoxylierte Alkohole, ethoxylierte Triglyceride, ethoxylierte Öle, Monoglyceride, Carboxylsäureester von Alkyl- oder Alkenylglycolen, C1-C40-Fettsäureester von Polyolen, C1-C40-Fettsäureether von Polyolen, C1-C40- Fettsäureester von Alkyl- oder Alkenylglycolen, Polyglycerinester, Polyglycerinester von C1-C40-Fettsäuren, Kohlenwasserstoffabgeleitete Ester, Zuckerester und -polyester, alkoxylierte Zuckerester und -polyester, ethoxylierte Carboxylsäureester von C1-C40-Fettsäuren, Sorbitan- oder Polysorbatester von Fettsäuren, ethoxylierte Sorbitanester von Fettsäuren, ethoxylierte Zuckerether von Fettsäuren, alkoxylierte Derivate von C1-C40-Fettsäureestern von C1-C40-Fettalkoholen, alkoxylierte Derivate von C1-C40-Fettsäureethern von C1-C40-Fettalkoholen, Polyethylenglycolether, Polyethylenglycolester, ethoxylierte Polysiloxane, Alkylglycoside, Alkanolamide, Aminoxide, Cetylphosphat, Kaliumcetylphosphat, Diethanolamincetylphosphat, Caboxylsäuren und deren Derivate, Sulfonsäurederivate, Schwefelsäurederivate, Phosphorsäurederivate, ethoxylierte Fettetherphosphate, Fettsäureamide, Acyllactylate, Alkylamidoalkylamine, Alkylamine, Alkylimidazoline, alkylsubstituierte Aminosäuren und Mischungen von diesen.

17. Kosmetisches Produkt nach einem der vorhergehenden Ansprüche, **dadurch gekennzeichnet, dass** das mindestens eine Elektrolyt ausgewählt ist aus der Gruppe enthaltend Alkalimetallsalze, Ammoniak, niedermolekulare Amine, Salze von di- oder trivalenten Kationen, Phosphatsalze und Mischungen von diesen, insbesondere aus der Gruppe enthaltend Natriumcitrat, Natriumchlorid, Kaliumcitrat, Natriumhexametaphosphat, Calciumchlorid, Calciumcarbonat und Mischungen von diesen.

18. Kosmetisches Produkt nach einem der vorhergehenden Ansprüche, **dadurch gekennzeichnet, dass** das mindestens eine Lösungsmittel und/oder die mindestens eine Trägersubstanz ausgewählt ist aus der Gruppe enthaltend Glycole, Glycerin, polare und unpolare Öle, Kohlenwasserstoffe, Ether, Ester, mittel- und langkettige Alkohole, alkoxylierte Alkohole, polyhydrierte Alkohole, Polyole und Mischungen von diesen, insbesondere aus der Gruppe enthaltend Propylenglycol, Dipropylenglycol, Ethylenglycol, Glycerin, Diglycerin, Diacetin, Triacetin, Isopropylpalmitat, Isododecan, Isohexadecan, Triglyceride, Mineralöl und Mischungen von diesen.

19. Kosmetisches Produkt nach einem der vorhergehenden Ansprüche, **dadurch gekennzeichnet, dass** das Produkt ausgewählt ist aus der Gruppe enthaltend Deodorants, jegliche deodorierende Präparate, Stifte, Sprays, Aerosole, Cremes, Sonnenschutzmittel, Aftershaves, Lotionen, Grundierungscremes und Make-ups.

20. Kosmetisches Produkt nach einem der vorhergehenden Ansprüche, **dadurch gekennzeichnet, dass** das Produkt einen Wassergehalt von 3 bis 40 Gew.-%, insbesondere von 5 bis 20 Gew.-%, enthält.

21. Kosmetisches Produkt nach einem der vorhergehenden Ansprüche, **dadurch gekennzeichnet, dass** der schweißabsorbierende Komplex einen Massenanteil von 0,05 bis 99 Gew.-%, insbesondere 0,1 bis 10 Gew.-%, vorzugsweise 0,25 bis 5 Gew.-% im Produkt aufweist.

22. Kosmetisches Produkt nach einem der vorhergehenden Ansprüche, weiterhin umfassend Hilfsstoffe und/oder Wirkstoffe ausgewählt aus der Gruppe enthaltend Pigmente, Farbstoffe, Antioxidantien, Konservierungsmittel, andere feuchtigkeitsspeichernde Substanzen, Weichmacher, Duftstoffe, Stabilisatoren, Zellturnover-Promotoren, Zellproliferations-Stimulatoren, entzündungshemmende Agenzien, antimikrobielle Agenzien, Hormonregulatoren, Enzyminhibitoren, UV-Absorber, Sonnenschutzstoffe und Mischungen von diesen.

23. Kosmetisches Produkt nach einem der vorhergehenden Ansprüche, **dadurch gekennzeichnet, dass** das Produkt ein Deodorant ist, das 0,1 bis 10 Gew.-% des schweißabsorbierenden Komplexes in einer Deodorantbasisformulierung enthält.

24. Kosmetisches Produkt nach Anspruch 23, wobei die Deodorantbasisformulierung 60 bis 90 %, insbesondere 65 bis 80 Gew.-% mindestens eines Lösungsmittels, 5 bis 30 %, insbesondere 10 bis 25 Gew.-% Wasser und 1 bis 20 %, insbesondere 5 bis 15 Gew.-% mindestens eines Gelierungsmittels enthält.

25. Kosmetisches Produkt nach einem der vorhergehenden Ansprüche, wobei die Schritte (I), (II) und/oder (III) bei einer Temperatur zwischen 50 und 100 °C, insbesondere zwischen 60 und 90 °C, vorzugsweise zwischen 70 und 80 C durchgeführt werden.

## Claims

1. A sweat-absorbing cosmetic product, said product containing
i) a base formulation, and
ii) a sweat-absorbing complex comprising
(a) 0.1 to 90 wt.-% of at least one water-absorbing component,
(b) 0.1 to 80 wt.-% of at least one surface-active agent,
(c) 0.001 to 20 wt.-% of at least one electrolyte, and
(d) 0 to 50 wt.-% of at least one solvent and/or at least one vehicle,
wherein the sweat-absorbing complex forms particles of a three-dimensional water-swellable network of said at least one water-absorbing component, which particles are at least partly coated by said at least one surface-active agent and are emulsified in the base formulation,
wherein the cosmetic product is obtainable by a method comprising the steps of:
(I) mixing the at least one water-absorbing component and the at least one electrolyte,
(II) adding the at least one surface-active agent with stirring and heat supply until a substantially homogeneous mixture is obtained,
(III) if included in the complex, adding the at least one solvent and/or the at least one vehicle and stirring until a substantially homogeneous mixture is obtained, and
(IV) mixing the thus obtained sweat-absorbing complex with the base formulation.

2. The cosmetic product according to claim 1, **characterized in that** said at least one electrolyte has a weight percentage in the sweat-absorbing complex of 0.01 to 20 wt.-%.

3. The cosmetic product according to claim 1 or 2, **characterized in that** the sweat-absorbing complex comprises
(a) 10 to 80 wt.-%, particularly 20 to 70 wt.-%, of said at least one water-absorbing component,
(b) 10 to 70 wt.-%, particularly 20 to 60 wt.-%, of said at least one surface-active agent,
(c) 0.05 to 15 wt.-%, particularly 0.1 to 10 wt.-%, of said at least one electrolyte, and
(d) 0 to 50 wt.-% of said at least one solvent and/or said at least one vehicle.

4. The cosmetic product according to any of the preceding claims, **characterized in that** at least part of said at least one electrolyte is incorporated in the interstices of the polymer network of said at least one water-absorbing component.

5. The cosmetic product according to any of the preceding claims, **characterized in that** said at least one water-absorbing component forms particles of a three-dimensional polymeric network when in contact with water.

6. The cosmetic product according to claim 5, **characterized in that** said at least one water-absorbing component forms particles with a mean particle size ranging from 0.1 to 450 µm, particularly from 10 to 200 µm, and preferably from 50 to 100 µm.

7. The cosmetic product according to any of the preceding claims, **characterized in that** said at least one water-absorbing component has a water absorption capacity of at least 20%, particularly at least 50%, based on the dry weight thereof.

8. The cosmetic product according to any of the preceding claims, **characterized in that** said at least one water-absorbing component is essentially insoluble in said at least one solvent and/or vehicle.

9. The cosmetic product according to any of the preceding claims, **characterized in that** said at least one water-absorbing component is selected from gums from vegetable or microbial biosynthesis, which essentially comprise repeats of monosaccharide units and have a molecular weight of at least 100,000 g/mol, particularly selected from the group including guar gum, guar derivatives, locust bean gum, cellulose gum, gum arabic, gum karaya, tragacanth, algae gums (comprising agar, carrageenan, furcellaran, and alginates), scleroglucan, tamarind seed gum, xanthan gum, dextran gum, gellan gum, propylene glycol alginate, derivatives and mixtures thereof.

10. The cosmetic product according to any of the preceding claims, **characterized in that** said at least one water-absorbing component is selected from polysaccharides of vegetable origin, essentially comprising repeats of monosaccharide units of hexoses and/or pentoses, particularly selected from the group including natural celluloses, natural fibers, cellulose derivatives, microcrystalline cellulose, carboxymethylcelluloses, methylcelluloses, hydroxyethylcelluloses, hydroxypropylcelluloses, hydroxypropylmethylcelluloses, pectins, maltodextrins, inulin, inulin derivatives, starch, starch derivatives, derivatives and mixtures thereof.

11. The cosmetic product according to any of the preceding claims, **characterized in that** said at least one water-absorbing component is selected from chemically modified polysaccharides, particularly from the group including cellulose derivatives, starch derivatives, pectin derivatives, starch/acrylamide/sodium acrylate graft copolymers (starch graft copolymers), and mixtures thereof.

12. The cosmetic product according to any of the preceding claims, **characterized in that** said at least one water-absorbing component is selected from polyacrylate-based synthetic polymers, particularly comprising repeats of acrylic acid, acrylamide, methacrylic acid, derivatives or mixtures thereof, particularly selected from the group including sodium polyacrylates, polyacrylamides, their copolymers, and/or mixtures thereof.

13. The cosmetic product according to any of the preceding claims, **characterized in that** said at least one water-absorbing component is selected from silicic acids and derivatives and modifications thereof, particularly from the group including polysilicic acids ((SiO₂)ₘ × nH₂O), silica (SiO₂), fumed silica, hydrated silica (SiO₂ × H₂O), silica gel, silicate salts and/or silicate esters.

14. The cosmetic product according to any of the preceding claims, **characterized in that** said at least one surface-active agent has a hydrophilic/lipophilic balance (HLB) ranging from 2 to 13, particularly from 3 to 11, especially from 3 to 9, preferably from 4 to 8.

15. The cosmetic product according to any of the preceding claims, **characterized in that** the complex has a mixture of surface-active agents having an effective weighted average hydrophilic/lipophilic balance (HLB) ranging from 2 to 13, particularly from 3 to 11, especially from 3 to 9, and preferably from 4 to 8.

16. The cosmetic product according to any of the preceding claims, **characterized in that** said at least one surface-active agent is a non-ionic, anionic, cationic or amphoteric compound or a mixture thereof, particularly selected from the group including fatty alcohols, ethoxylated alcohols, ethoxylated triglycerides, ethoxylated oils, monoglycerides, carboxylic esters of alkyl or alkenyl glycols, C₁-C₄₀ fatty acid esters of polyols, C₁-C₄₀ fatty acid ethers of polyols, C₁-C₄₀ fatty acid esters of alkyl or alkenyl glycols, polyglycerol esters, polyglycerol esters of C₁-C₄₀ fatty acids, hydrocarbon-derived esters, sugar esters and polyesters, alkoxylated sugar esters and polyesters, ethoxylated carboxylic esters of C₁-C₄₀ fatty acids, sorbitan or polysorbate esters of fatty acids, ethoxylated sorbitan esters of fatty acids, ethoxylated sugar ethers of fatty acids, alkoxylated derivatives of C₁-C₄₀ fatty acid esters of C₁-C₄₀ fatty alcohols, alkoxylated derivatives of C₁-C₄₀ fatty acid ethers of C₁-C₄₀ fatty alcohols, polyethylene glycol ethers, polyethylene glycol esters, ethoxylated polysiloxanes, alkyl glycosides, alkanolamides, amine oxides, cetyl phosphate, potassium cetyl phosphate, diethanolamine cetyl phosphate, carboxylic acids and derivatives thereof, sulfonic acid derivatives, sulfuric acid derivatives, phosphoric acid derivatives, ethoxylated fatty ether phosphates, fatty acid amides, acyl lactylates, alkylamidoalkylamines, alkylamines, alkylimidazolines, alkyl-substituted amino acids, and mixtures thereof.

17. The cosmetic product according to any of the preceding claims, **characterized in that** said at least one electrolyte is selected from the group including alkali metal salts, ammonia, low-molecular weight amines, salts of di- or trivalent cations, phosphate salts, and mixtures thereof, particularly from the group including sodium citrate, sodium chloride, potassium citrate, sodium hexametaphosphate, calcium chloride, calcium carbonate, and mixtures thereof.

18. The cosmetic product according to any of the preceding claims, **characterized in that** said at least one solvent and/or said at least one vehicle is selected from the group including glycols, glycerol, polar and non-polar oils, hydrocarbons, ethers, esters, medium- and long-chain alcohols, alkoxylated alcohols, polyhydric alcohols, polyols, and mixtures thereof, particularly from the group including propylene glycol, dipropylene glycol, ethylene glycol, glycerol, diglycerol, diacetin, triacetin, isopropyl palmitate, isododecane, isohexadecane, triglycerides, mineral oil, and mixtures thereof.

19. The cosmetic product according to any of the preceding claims, **characterized in that** the product is selected from the group including deodorants, any deodorizing preparations, sticks, sprays, aerosols, creams, sunscreens, after-shaves, lotions, foundation creams, and make-ups.

20. The cosmetic product according to any of the preceding claims, **characterized in that** the product has a water content of from 3 to 40 wt.-%, particularly from 5 to 20 wt.-%.

21. The cosmetic product according to any of the preceding claims, **characterized in that** the sweat-absorbing complex has a weight percentage in the product of from 0.05 to 99 wt.-%, particularly from 0.1 to 10 wt.-%, and preferably from 0.25 to 5 wt.-%.

22. The cosmetic product according to any of the preceding claims, further comprising auxiliary substances and/or active agents selected from the group including pigments, colorants, antioxidants, preservatives, other moisture-retaining substances, softeners, fragrances, stabilizers, cell turn-over promoters, cell proliferation stimulators, anti-inflammatory agents, antimicrobial agents, hormone regulators, enzyme inhibitors, UV absorbers, sunscreens, and mixtures thereof.

23. The cosmetic product according to any of the preceding claims, **characterized in that** the product is a deodorant containing 0.1 to 10 wt.-% of said sweat-absorbing complex in a deodorant base formulation.

24. The cosmetic product according to claim 23, wherein the deodorant base formulation includes 60 to 90%, particularly 65 to 80 wt.-% of at least one solvent, 5 to 30%, particularly 10 to 25 wt.-% water, and 1 to 20%, particularly 5 to 15 wt.-% of at least one gelling agent.

25. The method according to one of the preceding claims, wherein the steps (I), (II) and/or (III) are performed at a temperature between 50 and 100°C, particularly between 60 and 90°C, and preferably between 70 and 80°C.

## Revendications

1. Produit cosmétique absorbant la sueur contenant
i) une formulation de base et
ii) un complexe absorbant la sueur, comprenant
(a) 0,1 à 90 % en poids d'au moins un composant absorbant l'eau,
(b) 0,1 à 80 % en poids d'au moins un agent tensioactif,
(c) 0,001 à 20 % en poids d'au moins un électrolyte et
(d) 0 à 50 % en poids d'au moins un solvant et/ou d'au moins un excipient,
dans lequel le complexe absorbant la sueur forme des particules d'un réseau gonflable dans l'eau à trois dimensions du au moins un composant absorbant l'eau, qui sont revêtues au moins en partie avec le au moins un agent tensioactif et sont émulsifiées dans la formulation de base, moyennant quoi le produit cosmétique peut être préparé selon un procédé, qui comprend les étapes suivantes :
(I) mélanger le au moins un composant absorbant l'eau et le au moins un électrolyte,
(II) ajouter le au moins un agent tensioactif sous agitation et apport de chaleur, jusqu'à ce qu'on obtienne un mélange essentiellement homogène,
(III) dans la mesure où ils sont contenus dans le complexe, ajouter le au moins un solvant et/ou le au moins un excipient sous agitation, jusqu'à ce qu'on obtienne un mélange essentiellement homogène, et
(IV) mélanger le complexe absorbant la sueur ainsi obtenu à la formulation de base.

2. Produit cosmétique selon la revendication 1, **caractérisé en ce que** le au moins un électrolyte a une teneur en masse dans le complexe absorbant la sueur de 0,01 à 20 % en poids.

3. Produit cosmétique selon la revendication 1 ou 2, **caractérisé en ce que** le complexe absorbant la sueur comprend :
(a) 10 à 80 % en poids, notamment 20 à 70 % en poids du au moins un composant absorbant l'eau,
(b) 10 à 70 % en poids, notamment 20 à 60 % en poids du au moins un agent tensioactif,
(c) 0,05 à 15 % en poids, notamment 0,1 à 10 % en poids du au moins un électrolyte, et
(d) 0 à 50 % en poids du au moins un solvant et/ou du au moins un excipient.

4. Produit cosmétique selon l'une quelconque des revendications précédentes, **caractérisé en ce qu'**au moins des parties du au moins un électrolyte sont incorporées dans les interstices du réseau tridimensionnel du au moins un composant absorbant l'eau.

5. Produit cosmétique selon l'une quelconque des revendications précédentes, **caractérisé en ce que** le au moins un composant absorbant l'eau forme des particules d'un réseau polymère à trois dimensions lorsqu'il est en contact avec l'eau.

6. Produit cosmétique selon la revendication 5, **caractérisé en ce que** le au moins un composant absorbant l'eau forme des particules d'une granulométrie moyenne dans une plage de 0,1 à 450 µm, notamment de 10 à 200 µm, et de préférence de 50 à 100 µm.

7. Produit cosmétique selon l'une quelconque des revendications précédentes, **caractérisé en ce que** le au moins un composant absorbant l'eau a une capacité d'absorption d'eau d'au moins 20 %, notamment d'au moins 50 % par rapport au poids sec.

8. Produit cosmétique selon l'une quelconque des revendications précédentes, **caractérisé en ce que** le au moins un composant absorbant l'eau est essentiellement insoluble dans le au moins un solvant et/ou l'excipient.

9. Produit cosmétique selon l'une quelconque des revendications précédentes, **caractérisé en ce que** le au moins un composant absorbant l'eau est sélectionné parmi les gommes issues de biosynthèse végétale ou microbienne, qui comprennent essentiellement des répétitions d'unités monosaccharides et ont un poids moléculaire d'au moins 100.000 g/mole, et notamment sélectionné parmi le groupe comprenant la gomme de guar, les dérivés de guar, la farine de graines de caroube, la gomme de cellulose, la gomme arabique, la gomme karaya, le traganthe, les gommes d'algues (comprenant l'agar, le carraghénane, le furcellarane, et les alginates), le scléroglucane, la farine de graines de tamarin, le xanthane, le dextrane, le gellane, le propylène glycol alginate, les dérivés et les mélanges d'entre eux.

10. Produit cosmétique selon l'une quelconque des revendications précédentes, **caractérisé en ce que** le au moins un composant absorbant l'eau est sélectionné parmi les polysaccharides d'origine végétale, qui comprennent essentiellement une répétition d'unités monosaccharides d'hexoses et/ou de pentoses, notamment parmi le groupe comprenant les celluloses naturelles, les fibres naturelles, les dérivés de cellulose, la cellulose microcristalline, les carboxyméthylcelluloses, les méthylcelluloses, les hydroxyéthylcelluloses, les hydroxypropylcelluloses, les hydroxypropylméthylcelluloses, les pectines, les maltodextrines, l'inuline, les dérivés d'inuline, l'amidon, les dérivés d'amidon, les dérivés et les mélanges d'entre eux.

11. Produit cosmétique selon l'une quelconque des revendications précédentes, **caractérisé en ce que** le au moins un composant absorbant l'eau est sélectionné parmi les polysaccharides chimiquement modifiés, notamment parmi le groupe comprenant les dérivés de cellulose, les dérivés d'amidon, les dérivés de pectine, les copolymères greffés amidon/acrylamide/sodium acrylate (copolymères greffés d'amidon) et les mélanges d'entre eux.

12. Produit cosmétique selon l'une quelconque des revendications précédentes, **caractérisé en ce que** le au moins un composant absorbant l'eau est sélectionné parmi les polymères synthétiques à base de polyacrylate, qui comprennent notamment des répétitions d'acide acrylique, d'acrylamide, d'acide méthacrylique, des dérivés ou des mélanges d'entre eux, qui sont choisis notamment parmi le groupe comprenant le polyacrylate de sodium, le polyacrylamide, leurs copolymères et/ou des mélanges d'entre eux.

13. Produit cosmétique selon l'une quelconque des revendications précédentes, **caractérisé en ce que** le au moins un composant absorbant l'eau est sélectionné parmi les acides siliciques, leurs dérivés et leurs modifications, notamment parmi le groupe comprenant les acides polysiliciques
((SiO₂)ₘ x nH₂O), l'anhydride d'acide silicique (SiO₂), la silice pyrogénique, l'acide silicique hydraté (SiO₂ x H₂O), le gel de silice, les sels de silicate et/ou les esters de silicate.

14. Produit cosmétique selon l'une quelconque des revendications précédentes, **caractérisé en ce que** le au moins un agent tensioactif a une balance hydrophile/hydrophobe (HLB) dans une gamme de 2 à 13, notamment de 3 à 11, plus particulièrement de 3 à 9 et de préférence de 4 à 8.

15. Produit cosmétique selon l'une quelconque des revendications précédentes, **caractérisé en ce que** le complexe comprend un mélange d'agents tensioactifs, qui a une balance hydrophile/hydrophobe (HLB) effective de moyenne pondérée dans une gamme de 2 à 13, notamment de 3 à 11, plus particulièrement de 3 à 9 et de préférence de 4 à 8.

16. Produit cosmétique selon l'une quelconque des revendications précédentes, **caractérisé en ce que** le au moins un agent tensioactif est un composé non-ionique, anionique, cationique ou amphotère ou un mélange d'entre eux, et est notamment choisi parmi le groupe comprenant les alcools gras, les alcools éthoxylés, les triglycérides éthoxylés, les huiles éthoxylées, les monoglycérides, les esters d'acides carboxyliques et d'alkyl- ou alcénylglycols, les esters d'acides gras en C₁-C₄₀ et de polyols, les éthers d'acides gras en C₁-C₄₀ et de polyols, les esters d'acides gras en C₁-C₄₀ et d'alkyl- ou alcénylglycols, les esters de polyglycérol, les esters de polyglycérol d'acides gras en C₁-C₄₀, les esters dérivés d'hydrocarbure, les esters et polyesters de sucre, les esters et polyesters de sucre alcoxylés, les esters carboxyliques éthoxylés d'acides gras en C₁-C₄₀, les esters de sorbitane ou de polysorbate d'acides gras, les esters de sorbitane d'acides gras éthoxylés, les éthers de sucre d'acide gras éthoxylés, les dérivés alcoxylés des esters d'acides gras en C₁-C₄₀ et d'alcools gras en C₁-C₄₀, les dérivés alcoxylés des éthers d'acides gras en C₁-C₄₀ et d'alcools gras en C₁-C₄₀, les éthers de polyéthylène glycol, les esters de polyéthylène glycol, les polysiloxanes éthoxylés, les alkylglycosides, les alcanolamides, les amine oxydes, le cétylphosphate, le potassium cétyl phosphate, le diéthanolamine cétyl phosphate, les acides carboxyliques et les dérivés d'entre eux, les dérivés d'acide sulfonique, les dérivés d'acide sulfurique, les dérivés d'acide phosphorique, les phosphates d'éthers gras éthoxylés, les amides d'acide gras, les acyl lactylates, les alkylamidoalkylamines, les alkylamines, les alkylimidazolines, les acides aminés alkyl-substitués, et des mélanges d'entre eux.

17. Produit cosmétique selon l'une quelconque des revendications précédentes, **caractérisé en ce que** le au moins un électrolyte est choisi parmi le groupe comprenant les sels de métal alcalin, l'ammoniaque, les amines à bas poids moléculaire, les sels de cations di- ou trivalents, les sels de phosphate et des mélanges d'entre eux, notamment parmi le groupe comprenant le citrate de sodium, le chlorure de sodium, le citrate de potassium, l'hexamétaphosphate de sodium, le chlorure de calcium, le carbonate de calcium et les mélanges d'entre eux.

18. Produit cosmétique selon l'une quelconque des revendications précédentes, **caractérisé en ce que** le au moins un solvant et/ou le au moins un excipient est(sont) choisi(s) parmi le groupe comprenant les glycols, le glycérol, les huiles polaires et non polaires, les hydrocarbures, les éthers, les esters, les alcools à moyenne et longue chaîne, les alcools alcoxylés, les alcools polyhydriques, les polyols, et les mélanges d'entre eux, notamment dans le groupe comprenant le propylène glycol, le dipropylène glycol, l'éthylène glycol, le glycérol, le diglycérol, la diacétine, la triacétine, l'isopropyl palmitate, l'isododécane, l'isohexadécane, les triglycérides, l'huile minérale et les mélanges d'entre eux.

19. Produit cosmétique selon l'une quelconque des revendications précédentes, **caractérisé en ce que** le produit est sélectionné parmi le groupe comprenant les déodorants, toutes préparations déodorantes, les sticks, les sprays, les aérosols, les crèmes, les agents de protection solaire, les après-rasage, les lotions, les crèmes de fond de teint et les fards.

20. Produit cosmétique selon l'une quelconque des revendications précédentes, **caractérisé en ce que** le produit a une teneur en eau de 3 à 40 % en poids, notamment de 5 à 20 % en poids.

21. Produit cosmétique selon l'une quelconque des revendications précédentes, **caractérisé en ce que** le complexe absorbant la sueur a une teneur en masse dans le produit de 0,05 à 99 % en poids, notamment de 0,1 à 10 % en poids, et de préférence de 0,25 à 5 % en poids.

22. Produit cosmétique selon l'une quelconque des revendications précédentes, comprenant en plus des substances auxiliaires et/ou des principes actifs choisis parmi le groupe comprenant les pigments, les colorants, les antioxydants, les conservateurs, les autres substances retenant l'humidité, les adoucissants, les essences aromatiques, les stabilisants, les promoteurs de régénération des cellules, les stimulateurs de prolifération cellulaire, les agents anti-inflammatoires, les agents antimicrobiens, les régulateurs hormonaux, les inhibiteurs d'enzyme, les absorbeurs UV, les agents de protection solaire et les mélanges d'entre eux.

23. Produit cosmétique selon l'une quelconque des revendications précédentes, **caractérisé en ce que** le produit est un déodorant qui contient 0,1 à 10 % en poids du complexe absorbant la sueur dans une formulation de base de déodorant.

24. Produit cosmétique selon la revendication 23, dans lequel la formulation de base de déodorant contient 60 à 90 %, notamment 65 à 80 % en poids d'au moins un solvant, 5 à 30 %, notamment 10 à 25 % en poids d'eau et 1 à 20 %, notamment 5 à 15 % en poids d'au moins un agent gélifiant.

25. Produit cosmétique selon l'une quelconque des revendications précédentes, dans lequel les étapes (I), (II) et/ou (III) sont exécutées à une température comprise entre 50 et 100 °C, notamment entre 60 et 90 °C et de préférence entre 70 et 80 °C.
